Europäisches Patentamt

European Patent Office

Office européen des brevets

(19)

(11) Veröffentlichungsnummer : **0 082 447**
**B1**

(12) EUROPÄISCHE PATENTSCHRIFT

(45) Veröffentlichungstag der Patentschrift :
28.08.85

(51) Int. Cl.⁴ : **C 07 C 41/34, C 07 C 41/06**

(21) Anmeldenummer : 82111500.3

(22) Anmeldetag : 11.12.82

(54) Verfahren zur Abtrennung von Methanol aus den bei der Verätherung von C4 bis C7-Isoolefinen mit Methanol anfallenden Reaktionsprodukten.

(30) Priorität : 22.12.81 DE 3150755

(43) Veröffentlichungstag der Anmeldung :
29.06.83 Patentblatt 83/26

(45) Bekanntmachung des Hinweises auf die Patenterteilung : 28.08.85 Patentblatt 85/35

(84) Benannte Vertragsstaaten :
AT BE CH DE FR GB IT LI NL SE

(56) Entgegenhaltungen :
EP-A- 0 043 478
FR-A- 2 293 411

(73) Patentinhaber : DEUTSCHE TEXACO AKTIENGE-
SELLSCHAFT
Überseering 40
D-2000 Hamburg 60 (DE)

(72) Erfinder : Osterburg, Günther
Buchenstrasse 1
D-4100 Duisburg 17 (DE)
Erfinder : Prezelj, Milan
Grillparzer Strasse 59
D-6000 Frankfurt/Main (DE)

(74) Vertreter : Schupfner, Gerhard et al
Patentanwälte Dipl.-Ing. Hans-Jürgen Müller
Dipl.-Chem.Dr.phil.nat. Gerhard Schupfner Dipl.-Ing.
Hans-Peter Gauger
Karlstrasse 5 D-2110 Buchholz in der Nordheide (DE)

EP 0 082 447 B1

# 0 082 447

**Beschreibung**

Die Erfindung bezieht sich auf ein Verfahren zur Auftrennung des bei der an sich bekannten Herstellung von unsymmetrischen Äthern durch Umsetzung von $C_4$ bis $C_7$-Isoolefinen mit Methanol in Gegenwart eines sauren Katalysators, vorzugsweise eines sauren Kationenaustauscherharzes, erhalten Reaktionsgemischs und Abtrennung des nicht umgesetzten Methanols mittels einer Wasserwäsche mit anschließender Abtrennung der organischen und wässrigen Phasen.

Vorzugsweise stammt das isoolefinhaltige Einsatzmaterial der Umsetzung von einem aus der Pyrolyse oder der katalytischen Krackanlage stammenden leichten Kohlenwasserstoff (KW)-Schnitt. Die reagierenden Komponenten sind insbesondere Isobuten und Isopenten, der angewendete Alkohol Methanol, die gebildeten Äther insbesondere Methyl-t-butyl-äther (MTBE) und Methyl-t-amyl-äther (TAME), wichtige Zusatzmittel zu Motortreibstoffen.

Das Verfahren zur Herstellung der Äther und die Auftrennung des ätherhaltigen Reaktionsgemischs mittels einer der Reaktorstufe direkt folgenden Waschstufe ist Gegenstand der DE-OS-2 547 380. Diese Sequenz der Aufbereitungsschritte, nämlich Wäsche vor der destillativen Auftrennung, hat den wesentlichen Vorteil, daß man bei der Methanoldosierung nicht festgelegt ist und die vollständige Heausnahme des Alkohols in jedem Falle in einem Verfahrensschritt vornimmt, ohne daß man dies mit der Kombination umständlicher Azeotropdestillationen bewerkstelligen muß, wozu gegebenenfalls noch weitere Kolonnen erforderlich sind.

Mit einer geeignet ausgelegten Waschkolonne, etwa einer konventionellen Extraktionskolonne mit 5 theoretischen Extraktionsstufen, Methanol/Wasser-Verhältnis etwa 1 : 10 bis 1 : 20, werden dabei alle Alkohole, also neben dem Methanol auch die als Nebenprodukt gebildeten tertiären Alkohole, insbesondere t-Butanol (TBA), aus dem Reaktionsgemisch extrahiert. Die Äther, z. B. MTBE, verbleiben dabei praktisch vollständig in der organischen Phase, da die Anwesenheit der im Einsatzmaterial enthaltenen, nicht umgesetzten Kohlenwasserstoffe die Ätherverteilung in diesem Sinne begünstigt.

Der hierbei erhaltene wässrige Extrakt aus dem Sumpf der Waschkolonne enthält außer Wasser etwa 5 bis 10 % Methanol, 0,5 bis 1 % MTBE und 0,3 bis 0,5 % TBA. Dieses Gemisch wird einer atmosphärischen Destillation unterworfen, um das Methanol zwecks Wiederverwendung zurückzugewinnen.

MTBE geht bei dieser Destillation als Azeotrop mit Methanol über Kopf, wird in den Reaktor zurückgeführt und bereitet in diesem Zusammenhang keine weiteren Schwierigkeiten.

Anders bei den tertiären Alkoholen und insbesondere TBA : Bei der Destillation des wässrigen Extraktes destilliert dieser Alkohol als Wasser-Azeotrop über und erhöht damit zunächst den Wassergehalt im Methanol-Rückführstrom.

Dieses mit dem Rückführstrom in den Reaktor eingeführte Wasser bewirkt seinerseits eine Verschiebung des Reaktionsablaufs der Isobuten-Umsetzung in Richtung der TBA-Bildung. Ein Anreicherungskreislauf mit zusätzlichem « Schneeballeffekt » wäre durch wiederholte Extraktion des TBA in der Wasserwäsche und anschließende Rückführung zum Reaktor geschlossen, da keine Ausschleusung über die gewaschene organische Phase stattfindet. Eine solche Anreicherung des TBA in dem Methanolkreislauf unterdrückt bereits nach kurzer Betriebszeit die MTBE-Synthese im Reaktor.

Der Erfindung liegt die Aufgabe zugrunde, ein verbessertes Verfahren zur Auftrennung des bei der Synthese von tert. Alkyläthern anfallenden Produktgemischs zu schaffen unter Anwendung einer dem Reaktor direkt nachgeschalteten Wasserwäsche, deren wässriger Extrakt dann destillativ aufgearbeitet wird. Die im wässrigen Extrakt der Waschkolonne anfallende Methanolmenge soll möglichst vollständig von den tertiären Alkoholen abgetrennt und wiederverwendet werden.

Diese Aufgabe wird gelöst durch ein Verfahren zur Abtrennung von Methanol aus den bei der Verätherung von $C_4$ bis $C_7$-Isoolefinen mit Methanol in einem Reaktor anfallenden Reaktionsprodukten durch eine dem Reaktor direkt nachgeschaltete Wasserwäsche in Gegenwart von inerten Kohlenwasserstoffen, wobei ein Methanol und tertiäre Alkohole enthaltender Alkoholanteil mit Wasser extrahiert wird, die wässrige Extraktlösung sich im Sumpf der Waschkolonne sammelt und von da aus in eine Destillationskolonne übergeleitet wird, in der die Alkohole und Wasser destillativ getrennt werden, und wobei mit Wasser bei einem Methanol/Wasser-Verhältnis von 1 : 1,5 bis 1 : 10 bei einer Temperatur von 20 °C bis 60 °C, vorgugsweise bei einem Methanol/Wasser-Verhältnis von 1 : 2 bis 1 : 8, insbesondere 1 : 2 bis 1 : 5 und einer Temperatur von 40 °C bis 50 °C, der Alkoholanteil extrahiert, der anfallende wässrige Extrakt sodann zur Abtrennung des Alkoholanteils destilliert und aus der Destillationskolonne Methanol über Kopf abgezogen wird und von einem Boden mit hoher Konzentration an tertiären Alkoholen diese als Seitenstrom abgezogen und in die Waschkolonne zurückgeführt werden.

Hierbei werden somit bei der Destillation des aus der Waschkolonne abgezogenen wässrigen Extrakts die tertiären Alkohole mittels Seitenabzugs aus der Methanol/Wasser-Kolonne entfernt. Eine Kontrollvorrichtung steuert die Menge des Seitenabzugs so, daß am Kopf der Kolonne im wesentlichen kein tertiärer Alkohol mehr austritt. Dieser Seitenabzug, der der Wasser/Alkohol-Trennkolonne von einem Boden mit hoher Anreicherung an tertiären Alkoholen entnommen wird, enthält außer den tertiären Alkoholen, insbesondere TBA, auch noch beträchtliche Mengen an Methanol (beispielsweise Zusammensetzung : 40 % TBA, 50 % Methanol and 10 % Wasser). Diese Methanolmenge kann bis zu 20 % des gesamten aus dem Reaktionsgemisch in der Wäsche zurückgewonnenen Methanols betragen.

2

Solche Methanolmengen können in einer wirtschaftlich betriebenen Produktionsanlage nicht in Form eines Nebenproduktes verlorengehen. Man kann dieses zwar entweder als Slop verfeuern oder dem MTBE-Produkt beimischen, doch ist dieses unwirtschaftlich bzw. mit einer unerwünschten Verschlechterung der MTBE-Fertigproduktqualität verbunden. Dieser Abzugsstrom aus der Methanol/Wasser-Kolonne wird erfindungsgemäß in die Waschkolonne zurückgeführt.

Aus dem Reaktionsgemisch der Äthersynthese werden erfindungsgemäß mit einer geeignet ausgelegten Waschkolonne, insbesondere einer unpulsierten Siebbodenextraktionskolonne, und einem Methanol/Wasser-Verhältnis von bis herab zu 1 : 2 das Methanol quantitativ, der TBA jedoch entsprechend seiner Konzentration im Reaktionsgemisch und dem durch die Extraktionstemperatur vorgegebenen Verteilungsfaktor nur teilweise aus dem Reaktionsgemisch extrahiert. Der nicht extrahierte TBA verbleibt in der MTBE/KW-Phase und verläßt den Prozeß als eine Komponente des MTBE-Produktes.

Hierbei ist es nicht erforderlich, daß der aus der Methanol/Wasser-Trennkolonne abzuziehende, Methanol und Wasser sowie die tertiären Alkohole enthaltende Seitenstrom in einer zweiten Destillationskolonne aufgearbeitet wird. Dieser Seitenstrom kann vielmehr überraschenderweise wieder in die Waschkolonne zurückgeführt werden. In der Regel wird er einfach dem Reaktorausgangsgemisch zugemischt.

Durch die Rückführung des Seitenstroms erhöht sich die Konzentration an tertiären Alkoholen in dem die Waschkolonne verlassenden MTBE/KW-Gemisch. Dieser Vorgang der Konzentrationserhöhung wiederholt sich so lange, bis die in dem MTBE/KW-Gemisch am Kopf der Waschkolonne enthaltene Menge an tertiären Alkoholen gleich der Menge ist, die bei der Verätherungsreaktion als Nebenprodukt entstanden ist und mit den anderen Reaktionsprodukten in die Waschkolonne eingebracht wird. Bei einer konstanten Menge an tertiären Alkoholen und gleichbleibender Wassermenge stellt sich demnach ein konstanter Kreislauf an tertiären Alkoholen zwischen Extraktor und Methanolkolonne ein. Steigt oder fällt der TBA-Anfall bei der Synthese bzw. ändert sich die Waschwassermenge, so wird sich die TBA-Verteilung und damit der TBA-Kreislauf in entsprechendem Maße verändern.

Ein hohes Wasserangebot im Verhältnis zum Raffinat begrenzt die Anwendung des Verfahrens der vorliegenden Erfindung, weil mit mehr Wasser mehr TBA extrahiert wird und somit der Austrag über die Raffinatphase zunehmend unwirtschaftlicher und letzlich nicht mehr tragbar ist. Eine Begrenzung auf ein Mindestverhältnis von Raffinat zu Wasser von 2 : 1 ist für TBA realistisch und unabhängig von den Bedingungen der Methanolextraktion physikalisch begründet. Das maximale Verhältnis von Raffinat zu Wasser ist für die praktische Anwendbarkeit des vorliegenden Verfahrens nicht begrenzt, weil mit wenig Wasser nur wenig TBA extrahiert wird. Für eine obere Begrenzung des Verhältnisses lassen sich beim MTBE-Verfahren lediglich Gründe anführen, die vor der TBA-Extraktion liegen. Wesentlich ist hierbei die Frage, mit welchem Isobutengehalt ein $C_4$-Schnitt noch wirtschaftlich in eine MTBE-Synthese eingesetzt und aufgearbeitet werden kann. So ergibt die Umsetzung eines $C_4$-Schnitts mit einem Isobutengehalt von 12 % oder 7 % bei 95 % igem Umsatz und einem Methanolüberschuß von 20 Mol % und einem Methanol/Wasser-Verhältnis von 1 : 1,5 ein.

Verhältnis Raffinat zu Wasser von 55 : 1 oder 87 : 1, d. h. nur ein unrealistisch geringer Isobutengehalt vermag das Verhältnis von Raffinat zu Wasser noch weiter zu erhöhen. Als obere Grenze für das Raffinat/Wasser-Verhältnis kann somit aus praktischen Erwägungen 80 : 1, vorzugsweise 60 : 1 angesetzt werden.

Ebenso wie bei dem beschriebene einstufigen Verfahren zur Herstellung von MTBE ist das erfindungsgemäße Verfahren zur Auftrennung des Reaktionsgemischs in einer weiteren Ausführungsform auch bei einem zweistufigen MTBE-Verfahren anwendbar, wie es beispielsweise aus DE-AS 27 06 465 bekannt ist.

Bei einem zweistufigen Verfahren wird das den ersten Reaktor verlassende Gemisch einer Destillationskolonne zugeleitet, in der nicht-reagierte Kohlenwasserstoffgemische am Kopf abgezogen werden, damit in einem zweiten Reaktor unter Zuleitung von Frischmethanol die Umsetzung der noch im Kohlenwasserstoffstrom enthaltenen Isoolefinrestmenge erfolgt. Entsprechend seiner Zusammensetzung enthält dieser am Kopf der Destillationskolonne abgezogene Kohlenwasserstof geringe (2 bis 3 %) azeotrope Methanolmengen. Im Sumpf der Kolonne fällt ein Gemisch aus MTBE, tertiären Alkoholen und die Restmenge des nicht umgesetzten Methanols an. Dieses Gemisch wird zum Zweck der Alkohol/Äther-Trennung der mit Wasser arbeitenden Extraktionskolonne zugeführt, nachdem es vorher mit dem kohlenwasserstoffreichen Produktstrom aus dem zweiten Reaktor vereinigt wurde. Die Anwesenheit von Kohlenwasserstoffen bei dem Waschvorgang verringert bekannterweise den Übergang von MTBE in die wässrige Phase und sorgt für die Gewinnung eines absolut methanolfreien Ätherprodukts, unabhängig davon, welche Methanolmengen den Reaktorstufen zugesetzt wurden. Wie bei den einstufigen MTBE-Herstellungsverfahren erfolgt erfindungsgemäß die destillative Auftrennung der wässrigen Phase unter Abzug eines Seitenstrom, der in die Waschzone zurückgeführt wird.

Auch in diesem Fall der zweistufigen MTBE-Synthese wird mit den erfindungsgemäßen Maßnahmen eine restlose Rückführung des nicht umgesetzten Methanols in den Syntheseprozeß erreicht.

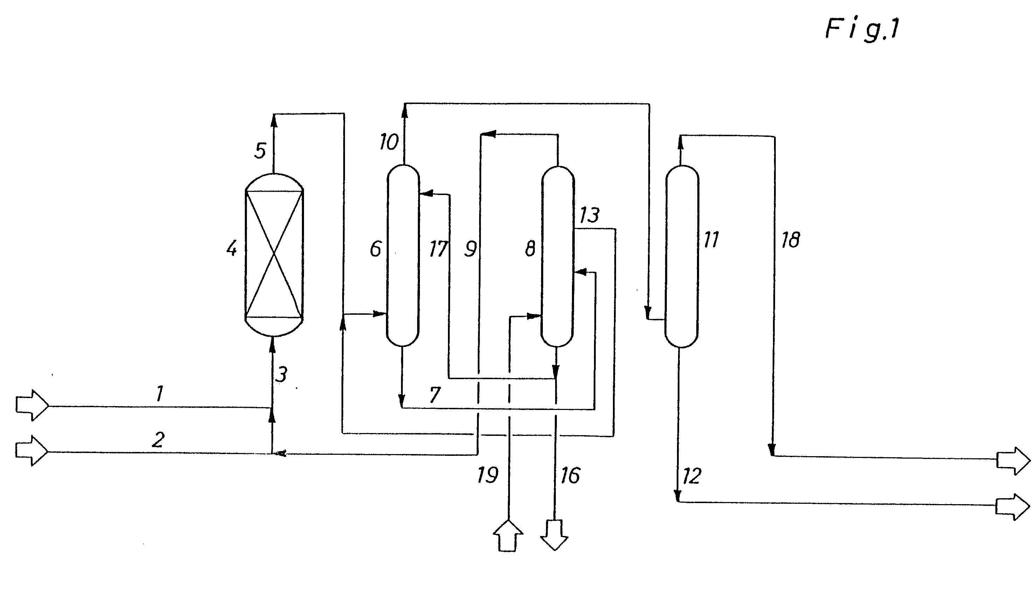

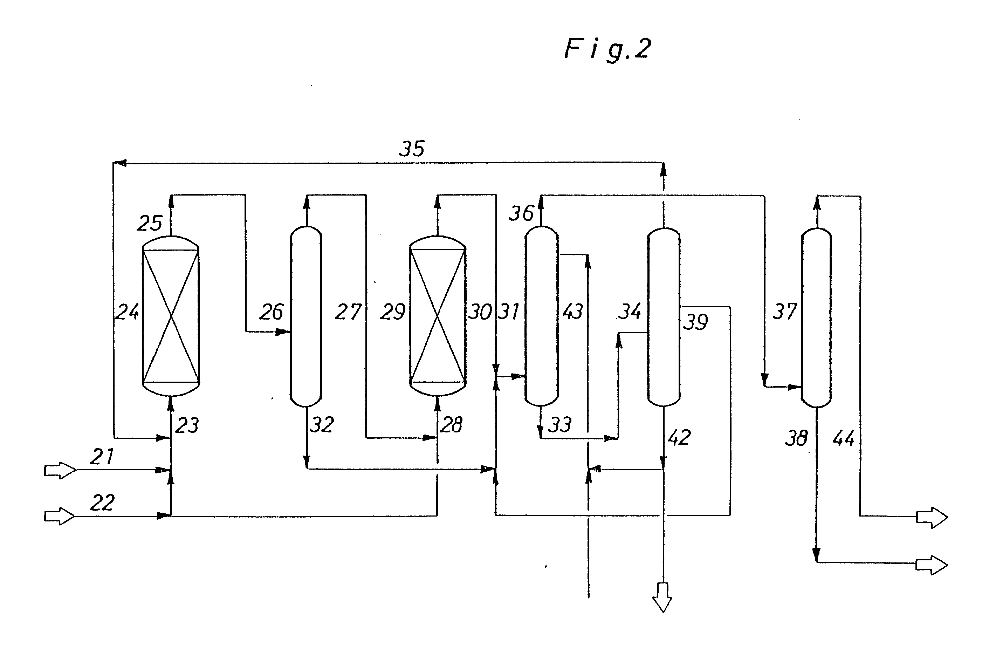

Im folgenden wird das erfindungsgemäße Verfahren, bezogen auf die Herstellung von MTBE, anhand der Figuren 1 und 2 beschrieben :

Figur 1   erläutert das einstufige Verfahren,

Figur 2   erläutert das zweistufige Verfahren.

0 082 447

Einstufen-Verfahren entsprechend Figur 1.

Ein Isobuten enthaltender $C_4$-Kohlenwasserstoffstrom aus Leitung 1, Frischmethanol aus Leitung 2 und ein MTBE-haltiger Methanolrückführstrom aus Leitung 9 werden gemeinsam über Leitung 3 in den Reaktor 4 gegeben. Das über die Leitung 5 den Reaktor 4 verlassende Reaktionsgemisch wird gemeinsam mit dem TBA-haltigen Seitenstrom aus Leitung 13 der unter Druck arbeitenden Extraktionskolonne 6 zugeführt. In dieser Extraktionskolonne wird das Methanol aus dem Reaktionsprodukt mit Wasser im Gegenstrom quantitativ ausgewaschen.

Im Sumpf der Extraktionskolonne 6 sammelt sich das Waschwasser, das neben dem gesamten Methanol in einer Konzentration von ca. 20 % außerdem noch ca. 1 % MTBE und die unter den Extraktionsbedingungen löslichen TBA-Anteile enthält. Über Leitung 7 wird dieses Waschwasser in die Methanol/Wasser-Destillationskolonne 8 geführt. In dieser Kolonne werden bei Normaldruck das Methanol mit dem im Wachwasser enthaltenen MTBE über Kopf abdestilliert, kondensiert und über die Leitung 9 als Methanolrückführstrom in den Reaktor zurückgeführt. Bei der Trennung Methanol/Wasser reichert sich der TBA in der Destillationskolonne 8 an, so daß an einem geeigneten Kolonnenboden ein TBA-Strom mit hoher TBA-Konzentration als Seitenstrom kontrolliert abgezogen werden kann, der über die Leitung 13 gemeinsam mit dem Reaktionsprodukt aus Leitung 5 wieder in die Extraktionskolonne 6 eingesetzt wird. Die typische Zusammensetzung eines solchen TBA-Seitenstromes beträgt etwa 40 % TBA, 50 % Methanol und 10 % Wasser. Das alkoholfreie und MTBE-freie Waschwasser wird von Sumpf der Destillationskolonne 8 über die Leitung 17 direkt wieder der Extraktionskolonne 6 zugeführt. Über Leitung 16 kann Wasser abgezogen werden. Leitung 19 dient der Zugabe von Wasser, wenn zusätzliches Wasser erforderlich ist.

Durch die Rückführung des TBA-Seitenstroms aus der Methanol/Wasser-Trennkolonne 8 in die Extraktionskolonne 6 wird der gesamte, durch die Umsetzung von Wasser mit Isobuten im Reaktor gebildete TBA mit dem dort ebenfalls gebildeten MTBE und den nicht umgesetzten bzw. inerten Kohlenwasserstoffen als Raffinatphase aus der Extraktionskolonne über Leitung 10 in die Destillationskolonne 11 gegeben. In der Destillationskolonne 11 werden die Kohlenwasserstoffe und MTBE getrennt. Am Kopf dieser Destillationskolonne wird ein methanol-, TBA-, und MTBE-freier $C_4$-Schnitt abgezogen und über Leitung 18 seiner Verwendung zugeführt. Im Sumpf der Kolonne 11 fällt methanolfreies MTBE gemeinsam mit dem gebildeten TBA an. Diese werden über die Leitung 12 ausgetragen.

Zweistufen-Verfahren entsprechend Figur 2.

Ein Isobuten enthaltender $C_4$-Kohlenwasserstoffstrom aus Leitung 21, Frischmethanol aus Leitung 22 und ein MTBE-haltiger Methanolrückführstrom aus Leitung 35 werden gemeinsam über Leitung 23 in den Reaktor 24 gegeben. Das über die Leitung 25 den Reaktor 24 verlassende Reaktionsgemisch wird der Destillationskolonne 26 zugeführt. Das Kopfprodukt der Destillationskolonne 26, ein $C_4$-Kohlenwasserstoffgemisch mit einem Restgehalt an nicht umgesetztem Isobuten, wird mit Leitung 27 abgezogen und zusammen mit Frischmethanol aus Leitung 28 in den Reaktor 29 eingeleitet, wo die Umsetzung des restlichen Isobutens erfolgt. Das über Leitung 30 den Reaktor 29 verlassende Reaktionsgemisch, bestehend aus nicht umsetzbaren $C_4$-Kohlenwasserstoffen, Äther und Alkohol, wird gemeinsam mit dem TBA-haltigen Seitenstrom aus Leitung 39 und dem über Leitung 32 abgezogenen Bodenprodukt der Destillationskolonne 26 der unter Druck arbeitenden Extraktionskolonne 31 in deren unteren Teil zugeführt. In der Extraktionskolonne 31 wird das Methanol mit Wasser im Gegenstrom quantitativ ausgewaschen.

Im Sumpf der Extraktionskolonne 31 sammelt sich das Waschwasser, das außer Wasser etwa 20 Gew.% Methanol, etwa 1 Gew.% MTBE und die unter den Extraktionsbedingungen löslichen TBA-Anteile enthält. Dieses Waschwasser wird über Leitung 33 in die Destillationskolonne 34 geleitet, wo das Methanol über Kopf abgeht und als Methanolrückführstrom über Leitung 35 und Leitung 23 dem Reaktor 24 zugeleitet wird, um wieder an der Reaktion teilzunehmen. MTBE verläßt gemeinsam mit den Kohlenwasserstoff-Komponenten die Extraktionskolonne 31 am Kopf über Leitung 36 und strömt durch diese Leitung in die Destillationskolonne 37, wo der MTBE im Sumpf abgeschieden und durch Leitung 38 ausgetragen wird. An einem geeigneten Kolonnenboden der Destillationskolonne 34 mit hoher TBA-Konzentration, einige Böden oberhalb des Eintritts von Leitung 33 in die Kolonne, wird über Leitung 39 ein TBA-haltiger Seitenstrom abgezogen und zusammen mit den Reaktionsprodukten aus den Leitungen 30 und 32 wieder in die Extraktionskolonne 31 geleitet.

Die typische Zusammensetzung des Seitenstroms beträgt etwa 40 Gew.% TBA, etwa 50 Gew.% MeOH und etwa 10 Gew.% $H_2O$. Das im Sumpf der Destillationskolonne 34 anfallende Wasser wird durch Leitung 42 ausgetragen bzw. über Leitung 43 in die Waschkolonne 31 überführt.

Das den Kopf der Kolonne 37 verlassende Dampfgemisch enthält nur noch den Anteil der nicht umsetzbaren Komponenten des $C_4$-Kohlenwasserstoffgemisches und verläßt über Leitung 44 die Anlage. Über Leitung 38 wird aus dem Sumpf der Kolonne 37 methanolfreier MTBE gemeinsam mit TBA ausgetragen.

Die folgenden Beispiele erläutern die Erfindung :

Beispiel 1

34 618 Teile eines $C_4$-Kohlenwasserstoffstromes aus Leitung 1 mit folgender Zusammensetzung

4

| Isobutan | 311 Gew.-Teile |
|---|---|
| n-Butan | 2 041 Gew.-Teile |
| Buten-1 | 4 671 Gew.-Teile |
| cis-Buten-2 | 1 765 Gew -Teile |
| trans-Buten-2 | 2 699 Gew.-Teile |
| Isobuten | 10 380 Gew.-Teile |
| 1.3-Butadien | 12 733 Gew.-Teile |
| Wasser | 18 Gew.-Teile |

wurden zusammen mit 5 680 Teilen Frischmethanol aus Leitung 2 und 1 133 Teilen Methanol mit 60 Teilen MTBE aus Leitung 9 in den Reaktor 4 geführt, wo Isobuten und Methanol in Gegenwart des Katalysators Amberlyst 15 unter Bildung von MTBE miteinander reagierten.

Die Umsetzung im Reaktor erfolgte bei 70 °C und 12 bar. Der Isobutenumsatz betrug 96 %. Die nicht umgesetzten $C_4$-Kohlenwasserstoffe verließen den Reaktor unverändert. Der Methanolüberschuß betrug 20 Mol.%. Aus 18 Teilen des im Reaktoreinsatzprodukt enthaltenen Wassers (ca. 0,05 %) bildete sich im Reaktor durch Umsatz mit 56 Teilen Isobuten, entsprechend 0,6 % des eingesetzten Isobutens, 74 Teile TBA.

Das den Reaktor 4 verlassende Reaktionsgemisch hatte folgende Zusammensetzung :

| Inerte KW | 24 190 Teile |
|---|---|
| Isobuten | 415 Teile |
| Methanol | 1 133 Teile |
| TBA | 74 Teile |
| MTBE | 15 631 Teile |
| Crotyläther | 48 Teile |

In der Extraktionskolonne 6 wurde dieser Produktstrom gemeinsam mit dem Produkstrom aus Leitung 13 (15 Teile TBA, 21 Teile Methanol und 4 Teile Wasser) mit 4 728 Teilen Wasser im Gegenstrom bei 40 °C gewaschen.

Das Verhältnis Methanol : Wasser betrug in der Waschkolonne etwa 1 : 4. Durch einen Überdruck von 12 bar wurden die $C_4$-Kohlenwasserstoffe in flüssiger Form gehalten. Das Methanol löste sich quantitativ in der Extraktphase. MTBE löste sich durch seine Eigenlöslichkeit in Gegenwart von Kohlenwasserstoffen zu etwa 1 % in der Extraktphase. TBA löste sich unter diesen Extraktionsbedingungen nur begrenzt in der Extraktphase, so daß von insgesamt 89 Teilen TBA, die in die Extraktionskolonne eingebracht wurden, 74 Teile TBA mit dem Raffinatstrom ausgetragen wurden. Das Raffinat bestand aus :

24 190 Teilen inerte Kohlenwasserstoffe
  415 Teilen Isobuten
15 571 Teilen MTBE
   48 Teilen Crotyläther
   74 Teilen TBA
   60 Teilen Wasser

40 358 Teilen Raffinat (Raffinat : Wasser = 8,5 : 1)

und wurde in der Destillationskolonne 11 getrennt. Am Kopf 20 der Destillationskolonne wurden als Destillat

24 220 Teile inerte Kohlenwasserstoffe
   415 Teile Isobuten
    60 Teile Wasser

abgezogen, aus dem das nicht lösliche Wasser abgeschieden 25 und abgetrennt wurde.

Am Sumpf der Kolonne 11 wurden 15 571 Teile MTBE mit 74 Teilen TBA und 48 Teilen Crotyläther abgezogen.

Die Extraktphase wurde mit

4 672 Teile Wasser
1 154 Teile Methanol
   60 Teile MTBE
   15 Teile TBA

in die Methanol/Wasser-Trennkolonne 8 eingesetzt. Durch Destillation wurden die gelösten organischen Anteile aus dem Wasser ausgetrieben, so daß am Kopf der Kolonne 1133 Teile Methanol und 60 Teile MTBE abgezogen und in den Reaktor zurückgeführt werden konnten.

An einem Boden mit höchster TBA-Konzentration, dem Boden 28, wurde der mit der Extraktphase in die Trennkolonne 8 eingebrachte und gegenüber Methanol und Wasser angereicherte TBA als Seitenstrom abgezogen und wieder in die Extraktionskolonne 6 zurückgeführt.

Die Menge des Seitenstromes betrug :

15 Teile TBA
21 Teile Methanol
 4 Teile Wasser

Somit wurden von insgesamt 89 Teilen TBA 15 Teile = 17 % extrahiert, abgezogen und in die Extraktionskolonne 6 zurückgeführt.

### Beispiel 2

34 620 Teile eines $C_4$-Kohlenwasserstoffstromes aus Leitung 1 mit folgender Zusammensetzung

| | |
|---|---:|
| Isobutan | 311 Gew.-Teile |
| n-Butan | 2 041 Gew.-Teile |
| Buten-1 | 4 671 Gew.-Teile |
| cis-Buten-2 | 1 765 Gew.-Teile |
| trans-Buten-2 | 2 699 Gew.-Teile |
| Isobuten | 10 380 Gew.-Teile |
| 1.3-Butadien | 12 733 Gew.-Teile |
| Wasser | 20 Gew.-Teile |

wurden mit 5 767 Teilen Frischmethanol aus Leitung 2 und 4 590 Teilen Methanol + 230 Teilen MTBE aus Leitung 9 in den Reaktor 4 geführt.

Die Umsetzung im Reaktor erfolgte bei 70 °C und 12 bar. Der Isobutenumsatz betrug 97,3 %. Der Methanolüberschuß betrug 80 Mol.-%.

Aus 20 Teilen des im Reaktoreinsatzprodukt enthaltenen Wassers (ca. 0,05 %) bildeten sich mit 62 Teilen Isobuten, entsprechend 0,6 % des eingesetzten Isobutens, 82 Teile TBA.

Das Reaktorausgangsprodukt hatte folgende Zusammensetzung :

| | |
|---|---:|
| Inerte Kohlenwasserstoffe | 24 171 Teile |
| Isobuten | 277 Teile |
| Methanol | 4 590 Teile |
| TBA | 82 Teile |
| MTBE | 16 009 Teile |
| Crotyläther | 78 Teile |

Dieser Strom wurde gemeinsam mit dem TBA-Seitenstrom aus Leitung 13 (123 Teile TBA, 176 Teile Methanol und 33 Teile Wasser) mit 18 387 Teilen Wasser bei 40 °C im Gegenstrom gewaschen.

Das Verhältnis Methanol : Wasser betrug 1 : 4. Das Methanol wurde quantitativ ausgewaschen. MTBE löste sich zu 1 % in der Extraktphase. TBA löste sich begrenzt, den Extraktionsbedingungen entsprechend, so daß von 205 Teilen TBA, die in die Extraktionskolonne eingebracht wurden, 82 Teile TBA mit dem Raffinatstrom ausgetragen wurden.

Zusammensetzung der Raffinatphase :

24 171 Teile inerte Kohlenwasserstoffe
   277 Teile Isobuten
15 779 Teile MTBE
    78 Teile Crotyläther
    82 Teile TBA
    60 Teile Wasser
_____
40 447 Teile Raffinat (Raffinat : Wasser = 2,2 : 1)

In der Destillationskolonne 11 wurden am Kopf

24 220 Teile inerte Kohlenwasserstoffe
   277 Teile Isobuten
    60 Teile Wasser

abgezogen, aus denen das Wasser abgeschieden und abgetrennt wurde.

Am Sumpf wurden 15 779 Teile MTBE, 82 Teile TBA und 78 Teile Crotyläther abgezogen.

Als Extraktphase wurden

18 360 Teile Wasser
4 766 Teile Methanol
230 Teile MTBE
123 Teile TBA

in die Trennkolonne 8 eingesetzt. Als kondensiertes Kopfprodukt wurden an dieser Kolonne 4 590 Teile Methanol und 230 Teile MTBE abgezogen und über Leitung 9 wieder dem Reaktor zugeführt.

Vom Boden 28 der Kolonne 8 wurde ein TBA-Seitenstrom mit 123 Teilen TBA, 176 Teilen Methanol und 33 Teilen Wasser abgezogen und in die Extraktionskolonne 6 zurückgeführt.

Von insgesamt 205 Teilen TBA wurden somit 123 Teile = 60 % extrahiert, abgezogen und zurückgeführt.

### Beispiel 3

123 948 Teile eines $C_4$-Kohlenwasserstoffstromes aus Leitung 1 mit folgender Zusammensetzung

| | |
|---|---|
| $C_3$-Kohlenwasserstoffe | 3 448 Gew.-Teile |
| Isobutan | 35 749 Gew.-Teile |
| n-Butan | 16 462 Gew.-Teile |
| Buten-1 | 12 753 Gew.-Teile |
| cis-Buten-2 | 9 837 Gew.-Teile |
| trans-Buten-2 | 14 710 Gew.-Teile |
| Isobuten | 18 003 Gew.-Teile |
| $C_5$-Kohlenwasserstoffe (Pentane, Pentene) | 12 924 Gew.-Teile |
| Wasser | 62 Gew.-Teile |

wurden mit 10 289 Teilen Frischmethanol und 8 231 Teilen Methanol und 390 Teilen MTBE aus Leitung 9 in den Reaktor 4 geführt.

Die Umsetzung im Reaktor erfolgte bei 70 °C und 12 bar. Der Isobutenumsatz betrug 97 %. Der Methanolüberschuß betrug 80 %.

Aus 62 Teilen des im Reaktoreinsatzprodukt enthaltenen Wassers (ca. 0,05 %) bildeten sich mit 193 Teilen Isobuten, entsprechend 1,1 % des eingesetzten Isobutens, 255 Teile TBA.

Das Reaktorausgangsprodukt hatte folgende Zusammensetzung :

92 959 Teile inerte Kohlenwasserstoffe
540 Teile Isobuten
12 006 Teile $C_5$-Kohlenwasserstoffe (Pentane, Pentene)
8 231 Teile Methanol
255 Teile TBA
27 529 Teile MTBE
1 338 Teile TAME

Dieser Strom wurde gemeinsam mit dem TBA-Seitenstrom aus Leitung 13 (127 Teile TBA, 182 Teile Methanol und 34 Teile Wasser) mit 33 652 Teilen Wasser bei 40 °C im Gegenstrom gewaschen.

Das Verhältnis Methanol : Wasser betrug 1 : 4. Das Methanol wurde quantitativ ausgewaschen. MTBE löste sich zu 1 % in der Extraktphase. TBA löste sich begrenzt, den Extraktionsbedingungen entsprechend, so daß von 382 Teilen TBA, die in die Extraktionskolonne eingebracht wurden, 255 Teile TBA mit dem Raffinatstrom ausgetragen wurden.

Zusammensetzung der Raffinatphase

92 959 Teile inerte Kohlenwasserstoffe
540 Teile Isobuten
12 006 Teile $C_5$-Kohlenwasserstoffe (Pentane, Pentene)
255 Teile TBA
132 Teile Wasser
27 139 Teile MTBE
1 338 Teile TAME

134 369 Teile Raffinat (Raffinat : Wasser = 4,0 : 1)

In der Destillationskolonne 11 wurden am Kopf

104 819 Teile inerte Kohlenwasserstoffe (einschl. $C_5$-Kohlenwasserstoffe)
540 Teile Isobuten
132 Teile Wasser

7

abgezogen, aus denen das Wasser abgeschieden und abgetrennt wurde.

Am Sumpf wurden 27 139 Teile MTBE, 255 Teile TBA, 1 338 Teile TAME und 146 Teile $C_5$-Kohlenwasserstoffe erhalten.

Als Extraktphase wurden

33 554 Teile Wasser
8 413 Teile Methanol
390 Teile MTBE
127 Teile TBA

in die Trennkolonne 8 eingesetzt. Als kondensiertes Kopfprodukt wurden an dieser Kolonne 8 231 Teile Methanol und 390 Teile MTBE abgezogen und über Leitung 9 wieder dem Reaktor zugeführt.

Vom Boden 28 der Kolonne 8 wurde ein TBA-Seitenstrom mit 127 Teilen TBA, 182 Teilen Methanol und 34 Teilen Wasser abgezogen und in die Extraktionskolonne 6 zurückgeführt.

Von insgesamt 382 Teilen TBA wurden somit 127 Teile = 33 % extrahiert, abgezogen und zurückgeführt.

## Beispiel 4

34 620 Teile eines $C_4$-Kohlenwasserstoffstromes aus Leitung 1 mit folgender Zusammensetzung

| | |
|---|---|
| Isobutan | 311 Gew.-Teile |
| n-Butan | 2 041 Gew.-Teile |
| Buten-1 | 4 671 Gew.-Teile |
| cis-Buten-2 | 1 765 Gew.-Teile |
| trans-Buten-2 | 2 699 Gew.-Teile |
| Isobuten | 10 380 Gew.-Teile |
| 1,3-Butadien | 12 733 Gew.-Teile |
| Wasser | 20 Gew.-Teile |

wurden mit 5 738 Teilen Frischmethanol und 4 590 Teilen Methanol und 141 Teilen MTBE aus Leitung 9 in den Reaktor 4 geführt.

Die Umsetzung im Reaktor erfolgte bei 70 °C und 12 bar. Der Isobutenumsatz betrug 97,3 %. Der Methanolüberschuß betrug 80 Mol.-%.

Aus 20 Teilen des im Reaktoreinsatzprodukt enthaltenen Wassers (ca. 0,05 %) bildeten sich mit 62 Teilen Isobuten, entsprechend 0,6 % des eingesetzten Isobutens, 82 Teile TBA.

Das Reaktorausgangsprodukt hatte folgende Zusammensetzung :

| | |
|---|---|
| inerte KW | 24 171 Teile |
| Isobuten | 277 Teile |
| Methanol | 4 590 Teile |
| TBA | 82 Teile |
| MTBE | 15 920 Teile |
| Crotyläther | 78 Teile |

Dieser Strom wurde gemeinsam mit dem TBA-Seitenstrom aus Leitung 13 (38 Teile TBA, 54 Teile Methanol und 10 Teile Wasser) mit 9 300 Teilen Wasser bei 40 °C im Gegenstrom gewaschen.

Das Verhältnis Methanol : Wasser betrug 1 : 2. Das Methanol wurde quantitativ ausgewaschen. MTBE löste sich zu 1 % in der Extraktphase. TBA löste sich begrenzt, den Extraktionsbedingungen entsprechend, daß von 120 Teilen TBA, die in die Extraktionskolonne eingebracht wurden, 82 Teile TBA mit dem Raffinatstrom ausgetragen wurden.

Zusammensetzung der Raffinatphase :

| | |
|---|---|
| 24 171 Teile | inerte Kohlenwasserstoffe |
| 277 Teile | Isobuten |
| 15 779 Teile | MTBE |
| 78 Teile | Crotyläther |
| 82 Teile | TBA |
| 60 Teile | Wasser |

40 447 Teile Raffinat (Raffinat : Wasser = 4,3 : 1)

In der Destillationskolonne 11 wurden am Kopf

24 220 Teile inerte Kohlenwasserstoffe

# 0 082 447

277 Teile Isobuten
60 Teile Wasser

abgezogen, aus denen das Wasser abgeschieden und abgetrennt wurde. Am Sumpf wurden 15 779 Teile MTBE, 82 Teile TBA und 78 Teile Crotyläther abgezogen.

Als Extraktphase wurden

9 240 Teile Wasser
4 644 Teile Methanol
141 Teile MTBE
38 Teile TBA

in die Trennkolonne 8 eingesetzt. Als kondensiertes Kopfprodukt wurden an dieser Kolonne 4 590 Teile Methanol und 141 Teile MTBE abgezogen und über Leitung 9 wieder dem Reaktor zugeführt.

Vom Boden 28 der Kolonne 8 wurde ein TBA-Seitenstrom mit 38 Teilen TBA, 54 Teilen Methanol und 10 Teilen Wasser abgezogen und in die Extraktionskolonne 6 zurückgeführt.

Somit wurden von insgesamt 120 Teilen TBA 38 Teile = 32 % extrahiert, abgezogen und zurückgeführt.

## Patentansprüche

1. Verfahren zur Abtrennung von Methanol aus den bei der Verätherung von $C_4$ bis $C_7$-Isoolefinen mit Methanol in einem Reaktor anfallenden Reaktionsprodukten in einer dem Reaktor direkt nachgeschaltete Waschkolonne in Gegenwart von inerten Kohlenwasserstoffen, wobei ein Methanol und tertiäre Alkohole enthaltender Alkoholanteil bei einer Temperatur von 20° bis 60 °C mit überschüssigem Wasser extrahiert wird, das erhaltene Raffinat als Produktstrom abgezogen und der wäßrige Extrakt zur Abtrennung des Alkoholanteils destilliert und aus der Destillationskolonne Methanol über Kopf abgezogen wird, dadurch gekennzeichnet, daß mit Wasser bei einem Methanol/Wasser-Verhältnis von 1 : 1,5 bis 1 : 10 der Alkoholanteil extrahiert, der anfallende wäßrige Extrakt destilliert und von einem Boden der Destillationskolonne mit hoher Konzentration an tertiären Alkoholen diese als Seitenstrom abgezogen und in die Waschkolonne zurückgeführt werden.

2. Verfahren nach Anspruch 1, dadurch gekennzeichnet, daß mit Wasser bei einem Verhältnis von Raffinat zu Wasser von mindestens 2 : 1 der Alkoholanteil extrahiert wird.

3. Verfahren nach Anspruch 1 oder 2, dadurch gekennzeichnet, daß mit Wasser bei einem Methanol/Wasser-Verhältnis von 1 : 2 bis 1 : 8, insbesondere 1 : 2 bis 1 : 5, bei einer Temperatur von 40 °C bis 50 °C der Alkoholanteil extrahiert wird.

4. Verfahren nach einem der Ansprüche 1 bis 3, dadurch gekennzeichnet, daß bei einem zweistufigen Verätherungsprozess, bei dem zwischen den beiden Reaktoren eine unter den normalen Bedingungen der $C_4$ bis $C_7$-KW-Abtrennung arbeitende Destillationsstufe zwischengeschaltet ist, das aus Äther, Methanol und tertiärem Alkohol bestehende Sumpfprodukt dieser Destillationsstufe mit dem aus dem zweiten Reaktor austretenden, inerte Kohlenwasserstoffe enthaltenden Reaktionsprodukt vereinigt und mit diesem zusammen in der Waschkolonne mit Wasser gewaschen wird, der in der Waschkolonne anfallende wässrige Extrakt in der Destillationskolonne destilliert, dabei Methanol über Kopf und tertiärer Alkohol als Seitenstrom abgezogen und letzterer in die Waschkolonne zurückgeführt wird.

## Claims

1. A process for the separation of methanol from the reaction products obtained in a reactor during the etherification of $C_4$-$C_7$ isoolefins with methanol in a subsequent washing column directly connected with the reactor and in the presence of inert hydrocarbons, an alcohol portion containing methanol and tertiary alcohols being extracted with excess water at a temperature of 20 °C to 60 °C, the raffinate obtained being withdrawn as product stream, and the aqueous extract being distilled to separate the alcohol portion, and methanol being stripped overhead from the distillation column, characterized by extracting the alcohol portion with water at a methanol/water ratio of 1 : 1,5 to 1 : 10, distilling the aqueous extract obtained, and withdrawing the tertiary alcohols as a slipstream from a tray of the distillation column having a high concentration thereof and recycling them to the washing column.

2. Process according to claim 1, characterized by extracting the alcohol portion with water at a raffinate/water ratio of at least 2 : 1.

3. Process according to claim 1 or 2, characterized by extracting the alcohol portion with water at a methanol/water ratio of 1 : 2 to 1 : 8, particularly 1 : 2 to 1 : 5 at a temperature of between 40 °C and 50 °C.

4. Process according to any one of the claims 1 to 3, characterized by uniting in a two-stage etherification process, in which a distillation stage operating under the normal conditions of separating

9

## 0 082 447

$C_4$-$C_7$ hydrocarbons is placed between the two reactors, the sump product consisting of ether, methanol, and tertiary alcohol from this distillation stage with the reaction product leaving the second reactor and containing inert hydrocarbons, by washing it together therewith with water in the washing column, by distilling in the distillation column the aqueous extract obtained in the washing column, thereby withdrawing methanol overhead and tertiary alcohol as a slipstream, and recycling the latter one to the washing column.

### Revendications

1. Procédé pour la séparation de méthanol des produits réactionnels formés dans un réacteur au cours de l'éthérification des isooléfines $C_4$-$C_7$ avec du méthanol dans une colonne de lavage subséquente directement raccordée au réacteur et en présence d'hydrocarbures inertes, une portion d'alcool contenant du méthanol et des alcools tertiaires étant extraite avec de l'eau excédente à une température comprise entre 20 °C et 60 °C, le raffinat obtenu étant soutiré en tant que courant de produit, l'extrait aqueux étant distillé pour en séparer la portion d'alcool, et du méthanol étant soutiré par la tête de la colonne de distillation, caractérisé en ce qu'on effectue l'extraction de la portion d'alcool avec de l'eau à un rapport méthanol/eau compris entre 1 : 1,5 et 1 : 10, qu'on distille l'extrait aqueux obtenu, et qu'on soutire les alcools tertiaires comme courant latéral d'un plateau de la colonne de distillation ayant une haute concentration d'alcools tertiaires et qu'on les recycle dans la colonne de lavage.

2. Procédé selon la revendication 1, caractérisé en ce qu'on extrait la portion d'alcool avec de l'eau à un rapport raffinat/eau de 2 : 1 au minimum.

3. Procédé selon la revendication 1 ou 2, caractérisé en ce qu'on extrait la portion d'alcool avec de l'eau à un rapport méthanol/eau compris entre 1 : 2 et 1 : 8, particulièrement entre 1 : 2 et 1 : 5, à une température comprise entre 40 °C et 50 °C.

4. Procédé selon l'une quelconque des revendications 1 à 3, caractérisé en ce que dans un procédé d'éthérification à deux étapes dans lequel un étage de distillation travaillant sous les conditions normales de la séparation d'hydrocarbures $C_4$-$C_7$ est intercalé entre les deux réacteurs on unit le produit de fond de cette étape de distillation renfermant de l'éther, du méthanol et de l'alcool tertiaire avec le produit réactionnel quittant le deuxième réacteur et contenant des hydrocarbures inertes et qu'on les lave ensemble à l'eau dans la colonne de lavage, qu'on distille dans la colonne de distillation l'extrait aqueux obtenu dans la colonne de lavage, en soutirant du méthanol par la tête et de l'alcool tertiaire comme courant latéral et qu'on recycle ce dernier dans la colonne de lavage.

Fig.1

Fig.2

0 082 447